Europäisches Patentamt

European Patent Office

Office européen des brevets

(19)

Publication number: **0 302 965**
**A2**

# EUROPEAN PATENT APPLICATION

(21) Application number: **87115940.6**

(22) Date of filing: **30.10.87**

(51) Int. Cl.4: **C04B 38/00 , C12N 11/14**

(30) Priority: **11.08.87 US 84265**

(43) Date of publication of application:
**15.02.89 Bulletin 89/07**

(84) Designated Contracting States:
**AT BE CH DE ES FR GB GR IT LI LU NL SE**

(71) Applicant: **MANVILLE CORPORATION**
**Ken-Caryl Ranch**
**Denver Colorado 80217(US)**

(72) Inventor: **Chelland, Joseph John**
**6655 W. Glasgow**
**Littleton Colorado 80123(US)**

(74) Representative: **Grättinger, Günter**
**Wittelsbacherstrasse 5 Postfach 16 49**
**D-8130 Starnberg(DE)**

(54) **Novel porous extruded shape biocarrier materials.**

(57) An extruded shape diatomaceous earth biocarrier for biologically active substances (e.g. enzymes) and cells having a mean pore diameter of at least about 20 microns. The biocarrier is made from either calcined or flux-calcined diatomite having a particle size of about 10 microns or greater and a mean pore diameter of about 5 microns or greater. The inventive biocarrier has a greater mean pore diameter and hardness compared to traditional biocarrier materials.

EP 0 302 965 A2

## NOVEL POROUS, EXTRUDED SHAPE BIOCARRIER MATERIALS

### Field of the Invention

This invention relates to a process for making porous, biocarrier materials and more particularly, it relates to a process for making novel, porous, extruded shape diatomaceous earth materials useful as biocarriers for enzymes, cells, and the like. This invention also relates to novel, extruded shape diatomaceous earth biocarrier materials of controlled porosity. This invention further relates to novel, porous, extruded shape diatomaceous earth biocarrier materials having proteins, cells, and the like within their pore structure.

### Background of the Invention

The manufacture of porous inorganic materials, e.g. diatomaceous earth, glass, silica, wollastonite, aluminum oxide, and silicon carbide, for use as filter mediums, contact materials, catalyst carriers, catalyst supports, and the like is known. See, for example, U.S. Patent Nos. 2,686,161; 3,526,602; 4,253,990; and 4,325,844. The foregoing disclose that these materials can be made into a variety of shapes and forms such as powders, pellets, and spheres. U.S. 3,526,602 and 4,409,247 also disclose that glass, silica, alumina, wollastonite, silicon carbide, etc., can be shaped into cylinders, truncated cones, discs, and annuluses. The attachment or immobilization of enzymes, microbial cells and the like to porous siliceous materials is also known to those skilled in the art. See, for example, U.S. Patent Nos. 3,666,627; 4,226,938; 4,409,247; 4,425,434; and 4,581,338.

The porous siliceous materials are typically made by the process of first forming a mixture of a siliceous material such as diatomite, a binder, a solvent, and an organic burnout material. The mixture is then formed into the desired form, e.g. most typically a powder, a pellet, or a sphere, which is then dried and/or calcined. Proteins, cells, and the like can then be attached to the porous substrate by any of a variety of well known methods and techniques.

While such porous, siliceous materials have found some degrees of commercial acceptance, their use is limited due to the fact that their greatest mean pore diameter is 20 microns or less. This limitation in mean pore diameter affects the number and types of cells, proteins, and the like which can be attached to the carriers. For example, it has not been heretofore convenient to attach some types of whole microbial, mammalian, and plant cells to existing porous carriers because the average pore diameters of the carriers are either partially or totally inadequate.

Even when the porous carriers can at least partially accommodate whole cells, several disadvantages are encountered in the use of such carrier/cell systems. For example, if the pore diameters are too low and the cells are therefore restricted from entry insufficient numbers of cells will be immobilized. Additionally, if spaces within the pores are be too small, a decreased flow of oxygen and nutrients to the cells will result. This leads to lower yields of desired products produced by the cells. Furthermore, higher numbers of cells on the outer surface of the carrier means more cells will become more susceptible to sudden and abrupt changes in the environment, e.g. pH shifts, which have an overall deleterious effect on the cells and their continuous ability to carry out biochemical conversions. Finally, when the pore diameters are inadequate, the natural affinity of the cells for the siliceous material and thus their tendency for self-attachment or immobilization to the porous material is greatly reduced.

Apart from the foregoing cited disadvantages due to the rather small pore structure of traditional biocarriers, further disadvantages are encountered in connection with the biocarriers because of their high attrition when used in certain types of reactors such as fluidized beds. The strength of the biocarriers is also important because it affects their ability to be formed and maintained into complex shapes beyond pellets and spheres. Tubes, sheets, cones, rings, polygonal shapes and the like may be needed in some reactor designs where the use of pellets and spheres present drawbacks. For example, it has been reported that in column reactors and fluidized beds, that some siliceous carriers have tended to be weak and compressible and therefore have given rise to difficulties, e.g. by compaction in column use resulting in a build-up of back pressure. Furthermore, the pellets and spheres because of their sizes and shapes tend to be packed very densely together when utilized thereby impeding the flow of nutrients and reaction medium through the reactor column or bed.

What is needed, therefore, is a porous biocarrier that can more readily accommodate enzymes, whole cells, and the like and which is stronger and more attrition resistant than existing siliceous biocarriers.

## Brief Summary of the Invention

In one embodiment of the present invention, Applicant has discovered an economical and easy to practice process for making porous, extruded shape biocarrier materials which have mean pore diameters of 20 microns or greater and which are more abrasion resistant and stronger than heretofore available supports and biocarriers. Briefly, Applicants' inventive process comprises first forming an extrudable mixture of 20-70 wt% of calcined or flux-calcined diatomite (or the like) wherein the diatomite particles have an average pore diameter of a least about 5 microns and a particle size of at least about 10 microns; 5-30 wt% of an inorganic binder; 0-30 wt% of an organic burnout material; 1-15 wt% of an organic polymer processing aid; and 20-50 wt% solvent. The resulting mixture is then extruded to form an extrudate of the desired shape which is then dried or calcined. By starting with diatomite particles (or the like) of a certain pore diameter and particle size, Applicant has discovered that the inventive process readily allows for the production of extruded shape biocarriers of controlled porosity.

In another embodiment, Applicant provides an extruded shape diatomaceous earth biocarrier material of controlled porosity having a mean pore diameter of 20 microns or greater. The inventive material is more abrasion resistant and stronger than currently available diatomaceous earth biocarrier materials and also has a mean pore diameter of 20 microns or greater which is considerably greater than the mean pore diameters of traditional biocarriers. The latter fact is particularly important since the inventive biocarrier will be able to accommodate proteins, whole cells, and the like without the aforementioned difficulties encountered with less porous biocarrier materials.

In a further embodiment of the present invention, extruded shapes diatomaceous earth biocarriers are provided which have proteins, cells, and the like contained in the pore structures of the biocarrier. The mean pore structure of the biocarrier is at least 20 microns or greater. Because of the relatively large pore structure of the biocarrier, the biological system of proteins/cells attached to the biocarrier will be very efficient because the difficulties encountered with traditional biocarriers are overcome. For example, the greater pore structure will allow for higher diffusion of oxygen and essential nutrients to attached cells within the pores as well as creating an increased diffusion into the surrounding environment of biochemical conversion product and other extra cellular materials generated by the cells within the pore structures of the biocarrier. Furthermore, because of the improved inventive biocarrier with larger internal pore structures, a larger internal population or loading of proteins, cells, and the like is possible. This is significant because the attached cells, etc., will be less subject to sudden shifts in the surrounding environment, e.g. pH. Finally, the larger pore diameters allow for an easier "natural" or self-attachment of cells to the walls of the pore structures within the biocarrier.

Other features and aspects of the invention, as well as their benefits, will be made clear in the more detailed description of the invention which follows.

## Detailed Description of the Invention

The inventive process comprises first forming an extrudable mixture of the following ingredients at the indicated general and preferred levels (wt%, based upon the total weight of the extrudable mixture):

| Ingredient | General | Preferred |
|---|---|---|
| Calcined or Flux-Calcined Diatomite | 20-70 | 30-45 |
| Inorganic Binder | 5-30 | 5-10 |
| Organic Burnout Material | 0-30 | 0-20 |
| Organic Polymeric Processing Acid | 1-15 | 3-8 |
| Solvent | 20-50 | 30-40 |

3

Diatomite is a chalky sedimentary material composed of the skeletal remains of single celled aquatic water plants called diatoms. Many modern diatomite deposits were laid down by sedimentation in shallow waters years ago. Subsequent geologic uplift has raised these beds to positions where they can be mined by conventional methods. Deposits are found in numerous parts of the world, with one of the largest and purest deposits being located on the central California coast. In other locations, there are currently shallow bodies of water where diatomite deposition has occurred and/or is currently occurring. Such deposits are presently mined by dredging. A typical dry diatomite analysis is shown in Table I below.

TABLE I

| Component | Wt.% |
|---|---|
| $SiO_2$ (a) | 86.0 |
| $Al_2O_3$ | 3.6 |
| $Fe_2O_3$ | 1.3 |
| Group I Oxides | 1.2 |
| Group II Oxides | 1.1 |
| Other | 0.5 |
| Water | 3.0 |
| Loss on Ignition | 3.6 |
| Note: | |

(a) predominantly in amorphous form

Calcined diatomite is diatomite which is mined, dried, granulated, and passed through a kiln which is operated at a temperature in the range of about 1600°F to 2400°F. The calcination causes the diatomite particles to shrink and harden and, to a certain extent, to agglomerate themselves into larger clusters.

Flux calcined diatomite is produced by adding a flux to the diatomite. The flux can be added as a solution dissolved in a water spray or mixing water. Alternatively, dry flux powder can be incorporated into the mass of diatomite particles either during air conveying of the diatomite or by dry mixing of the flux and diatomite in conventional dry mixing devices such as tumblers. Normally there will be from about 3 to about 10 weight percent flux based on the weight of the dry diatomite. Typical fluxes include alkali metal salts such as sodium carbonate ("soda ash"), sodium chloride, sodium hydroxide, and sodium silicate.

Whatever calcined or flux-calcined diatomite is used in the present invention should have an average pore diameter of greater than about 5 microns and preferably greater than about 10 microns. The particle size should be at least about 10 microns and preferably at least about 25 microns.

Another component of the present invention is an inorganic binder. Generally any commercially available inorganic binder may be used in the present invention. Of course, it must have the requisite strength to bind with the mixture ingredients, especially the diatomite.

One class of inorganic binder which may be used in the present invention are clays. Examples include kaolin clays and bentonite clays. Kaolin clays, sometimes referred to as white or porcelain clays are a white-burning clay, which due to their great purity, have a high fusion point. Kaolin clays are also the most refractory of all clays.

Bentonite clays are a form of montmorillonite clays. Bentonite clays are hydrous alumina silicates normally containing significant portions of sodium, magnesium, and calcium oxides.

Another class of inorganic binders which can be used are monovalent silicates. Examples of monovalent silicates include but are not limited to sodium silicate and potassium silicate with sodium silicate preferred.

Another class of inorganic binders are glassy frits made by pre-fluxing silica with excess amounts of alkaline oxide materials and grinding it to a powder form.

Other inorganic binders which may be utilized include phosphoric acid based binders such as aluminum phosphate and colloidal suspensions including colloidal silica, colloidal alumina, and colloidal zirconia.

Suitable combinations of the above inorganic binders may be utilized. However, frit and clay based binder systems are presently preferred.

Suitable organic burnout materials for use in the present invention include but are not limited to starches, cellulose fibers, corn meal, and powdered carbons. Examples of the cellulose fibers include kraft fiber, wood fiber, cob meal, and straw fibers. Short fiber lengths are preferred for ease in mixing and extruding.

The organic polymeric processing aid utilized herein is one which imparts an extrudable consistency to the mixture. Typical of such processing aids are the cellulose ethers, e.g. carboxymethyl cellulose.

Any commercially available solvent can be used in the present invention which will solubilize the solid components of the mixture. These solvents may be organic or aqueous in nature, however, an aqueous solvent is presently preferred.

Examples of suitable organic solvents include but are not limited to kerosene, diesel fuels, alcohols, and molten waxes.

After the mixture of the solids, processing aid, and solvent is formed into the extrusion feed, it is extruded in conventional extrusion equipment through a die to form an extrudate in shaped form or from which individual pellets may be separated. Most commonly the extrudate is an elongated rod-like material of circular, oval, or square cross-sections. Circular cross-sections are preferred for pellets to minimize attrition in subsequent handling. The extruded section can be from 0.06 to several inches in width or diameter.

When pellets are made the extruded rod is commonly severed at intervals approximately equal to the diameter or width of the rod such that generally cylindrical or cubical pellets having approximately equal dimensions in all dimensions are formed. Conventional severing equipment such as wire knives can be used.

Subsequent tumbling procedures can be used to produce spheres from the rod extrudate.

Shaped forms for the biocarriers include but are not limited to cones, tubes, cylinders, discs, annuluses, flat sheets, polygons, and star shaped materials.

After the desired extruded shapes are formed, they are dried in conventional drying units such as continuous belt dryers or temperature and humidity controlled rooms. The drying time and temperature will vary depending upon the nature of the extruded shape. Preferably, though, the drying temperature will be in the range of from about 70° to 400° F, most preferably about 200° to 300° F. Preferably the drying time will be from about 10 to 30 minutes and most preferably from about 15 to 20 minutes for pellets and preferably about 2 to 24 hours and most preferably about 5 to 10 hours for larger extrusions.

When calcined, as with drying, the conditions will vary depending upon the extruded shape. Preferably, though, the extrusions are calcined or fired in calcining equipment such as a shuttle or rotary kiln at a temperature in the range of about 1600° to 2100° F for about 10 to 40 minutes, most preferably at about 1700° to 1900° F for about 20 to 30 minutes.

The calcining time at firing temperature will normally be at least about 10 minutes and more on the order of about 30 minutes for pellets and about 1 hour for larger extrusions.

Calcining in an oxygen containing atomosphere should continue until all the organic burnout material, if any is present, has been burned out of the extrusion leaving a highly porous composite of diatomite and inorganic binder.

The resulting porous, extruded shape diatomaceous earth biocarrier will generally have a mean pore diameter of about 20 microns or greater. Preferably, the mean pore diameter will in the range of about 20-50 microns and most preferably about 25-35 microns.

As disclosed herein earlier, the inventive biocarrier can be extruded in a variety of different shapes to fit particular engineering reactor designs, e.g. hollow tubes, discs, cones, rings, annuleses, flat sheets, polygons, and stars. Thus, one practicing the invention has great flexibility in arriving at the desired shape and mean pore diameter of the diatomaceous earth carrier.

A variety of cells and biologically active substances can be attached and/or immobilized in the pores of the inventive biocarrier material.

The term "biologically active substance" as used herein embraces proteinaceous substances (e.g. enzymes) and includes substances which are biologically active per se and those which are not, but which can be activated after immobilization to make them biologically active. In the latter case a biologically active substance can be treated with an inhibitor to deactivate it and then it can then be reactivated after immobilization.

As used herein, the term "proteinaceous substances" signifies proteins per se and substances having a proteinaceous component (e.g. glyco-proteins such as amyloglucosidase and lypo-proteins.)

Furthermore, it is to be understood that the term "biologically active substance" includes inter alia those substances capable of participating in specific interactions, such substances including, for example, substances of biological origin and those which act on living organisms. Substances of synthetic origin which can participate in reactions involving specific interactions analogous to those which can occur with naturally occurring substances are also embraced within the term "biologically active substance".

Cells can also be attached/immobilized within the pores of the inventive carrier as well. As used herein, the term cell includes but is not limited to microbial, mammalian, and plant cells.

Non-limiting examples of cells which can be attached/immobilized to the inventive biocarriers include bacteria, yeast, fungi, algae, pancreatic cells, and lymphocytes.

The biologically active substances and cells can be attached or immobilized within the pores of the inventive biocarrier by any method known to those skilled in the art. For example, one may use known coupling or cross-linking agents. Alternatively, one may sometimes immobilize cells by simply contacting the biocarrier with an aqueous suspension of cells to be immobilized thereon as there can be a natural attraction between the cell walls and the biocarrier as produced.

The following non-limiting example illustrates the pore control and larger pore diameters available with an inventive biocarrier versus conventional biocarriers.

## EXAMPLE

A series of non-inventive and inventive biocarrier materials were made according to the following formulas. Their resulting properties are also given. All formulas are given in parts by weight.

EP 0 302 965 A2

| | | Formula | | |
|---|---|---|---|---|
| | 1 | 2 | 3 | 4 |
| Material | (non-inventive) | (non-inventive) | (inventive) | (inventive) |
| Precipitated Silica, .022 micron particle size | 100 | | | |
| Natural diatomite, 7.5 micron particle/ 1.5 micron pores | | 100 | | |
| Flux Calcined Diatomite, 100 micron particle/23 micron pores | | | 100 | 100 |
| Burn out, – 60 mesh | 40 | 40 | 30 | |
| Burn out, 40 x 60 mesh | | | | 30 |
| Inorganic Binder | 25 | 25 | 20 | 20 |
| Processing Aid | 14 | 14 | 14 | 14 |
| Water | 100 | 100 | 100 | 100 |

| | Properties of Extrudate |
|---|---|

| | | | | |
|---|---|---|---|---|
| Fire Temp, °F | 1600 | 1900 | 1850 | 1850 |
| **Properties of Extrudate** | | | | |
| Median Pore Diameter, Microns | 0.05 | 3.0 | 22.5 | 33 |
| Total Pore Volume, cc/g | 1.0 | 1.0 | 0.75 | 0.7 |
| Hardness, Kg | 2.5 | 3.5 | 7 | 8 |

The foregoing data clearly show that the inventive biocarriers 3 and 4 have mean pore diameters which are significantly greater than the non-inventive biocarriers 1 and 2. Inventive biocarriers 3 and 4 also have higher hardness values than 1 and 2 do.

Therefore, the inventive biocarriers will be able to accommodate a wide range of biologically active substances and cells as well as be much more durable in their use compared to traditional, non-inventive

8

biocarriers.

Reasonable modifications and variations are possible from the foregoing disclosure without departing from either the spirit or scope of the present invention as defined in the claims.

**Claims**

1. A process for the production of an extruded shape biocarrier material having a mean pore diameter of greater than about 20 microns, said process comprising the steps of:

(a) forming an extrudable mixture comprising about:

(i) 20-70 wt% of a calcined or flux calcined diatomite wherein said diatomite particles have an average pore diameter of at least about 5 microns and a particle size at least about 10 microns;

(ii) 5-30 wt% inorganic binder;

(iii) 0-30 wt% organic burnout material;

(iv) 1-15 wt% organic polymeric processing aid; and

(v) 20-50 wt% solvent;

(b) extruding the mixture to form an extrudate of the desired shape; and

(c) drying or calcining said extrudate.

2. A process according to claim 1 wherein said extrudable mixture comprises about:

(i) about 30-45 wt% calcined or flux calcined diatomite wherein said diatomite particles have an average pore diameter of at least about 10 microns and a particle size of at least about 25 microns;

(ii) 5-10 wt% inorganic binder;

(iii) 0-20 wt% organic burnout material;

(iv) 3-8 wt% organic polymeric processing aid; and

(v) 30-40 wt% solvent.

3. A process accoding to claim 1 where the extruded shape is a hollow tube.

4. A process according to claim 1 where the extruded shape is a star.

5. An extruded shape diatomaceous earth biocarrier for biologically active substances and cells having a mean pore diameter of at least about 20 microns.

6. An extruded shape diatomaceous earth biocarrier for biologically active substances and cells having a mean pore diameter of at least 20 microns made by the process of claim 1.

7. A biocarrier according to claim 6 in the form of a hollow tube.

8. A biocarrier according to claim 6 in the form of a star.

9. An extruded shape, diatomaceous earth biocarrier having a pore diameter of at least about 20 microns and containing in the pores thereof at least one selected material seeded from the group consisting of biologically active substances and cells.

10. A biocarrier according to claim 9 in the form of pellet, a sphere, a hollow tube, or a star.